# EUROPEAN PATENT APPLICATION

(11) **EP 4 122 950 A1**
(43) Date of publication of application: **25.01.2023**
(21) Application number: 21187364.1
(22) Date of filing: 23.07.2021
(51) Int. Cl.: C07K 14/47

(54) **RECOMBINANT HOST CELLS AND METHODS FOR PRODUCING CASEIN PROTEINS**

(71) Applicant: Redbiotec AG, 8952 Schlieren (CH)
(72) Inventor: Droz, Xuan, 8952 Schlieren (CH); Bühlmann, Martin, 8952 Schlieren (CH); Tambasco Studart, Marina, 8952 Schlieren (CH); Schaub, Christian, 8952 Schlieren (CH); John, Corinne, 8952 Schlieren (CH)
(74) Representative: Schiweck Weinzierl Koch Patentanwälte Partnerschaft mbB

(57) **Abstract**

The present invention relates to a recombinant host cell genetically engineered to produce caseins and methods of producing caseins.

## Description

### FIELD OF INVENTION

The present invention relates to a recombinant host cell or organism genetically engineered to express at least one casein protein and to methods of producing caseins using the recombinant host cell or organism.

### BACKGROUND

Caseins are a family of related phosphoproteins (αS1, αS2, β, κ). These proteins are commonly found in mammalian milk, comprising about 80% of the proteins in cow's milk and between 20% and 60% of the proteins in human milk. Sheep and buffalo milk have a higher casein content than other types of milk with human milk having a particularly low casein content. Bovine milk is known to have four specific caseins, αS1-casein, αS2 - casein, β-casein, and κ-casein.

Casein has a wide variety of uses, from being a major component of cheese, to use as a food additive. The most common form of casein is sodium caseinate. In milk, casein undergoes phase separation to form colloidal casein micelles. As a food source, casein supplies amino acids, carbohydrates, and two essential elements, calcium and phosphorus.

Casein contains a high number of proline amino acids which hinder the formation of common secondary structural motifs of proteins. As a result, it has relatively little tertiary structure. It is relatively hydrophobic. It is found in milk as casein micelles, which show only limited resemblance with surfactant-type micelles in a sense that the hydrophilic parts reside at the surface and they are spherical. However, in sharp contrast to surfactant micelles, the interior of a casein micelle is highly hydrated. The caseins in the micelles are held together by calcium ions and hydrophobic interactions.

Methods of recombinantly producing milk proteins such as caseins are known, for example from WO2016/029193, however these methods generally suffer from limitations. To date, there is no ideal system which can overcome the expression limitations such as poor yield ranges and expression of milk protein micelles, both being important parameters in the field of cow-free milk and products thereof.

Thus, there remains a need to provide improved host organisms and methods of producing caseins.

### SUMMARY

The present invention addresses this need and provides a recombinant host cell or organism genetically engineered to express at least one casein protein, wherein the polynucleotide encoding the casein protein is integrated into the host cell genome or is maintained episomally and is linked to a heterologous secretion signal and to a promoter and is followed by a terminator sequence. Specifically, the host cell or organism can be genetically engineered to produce at least one, at least two, at least three or at least four different casein proteins.

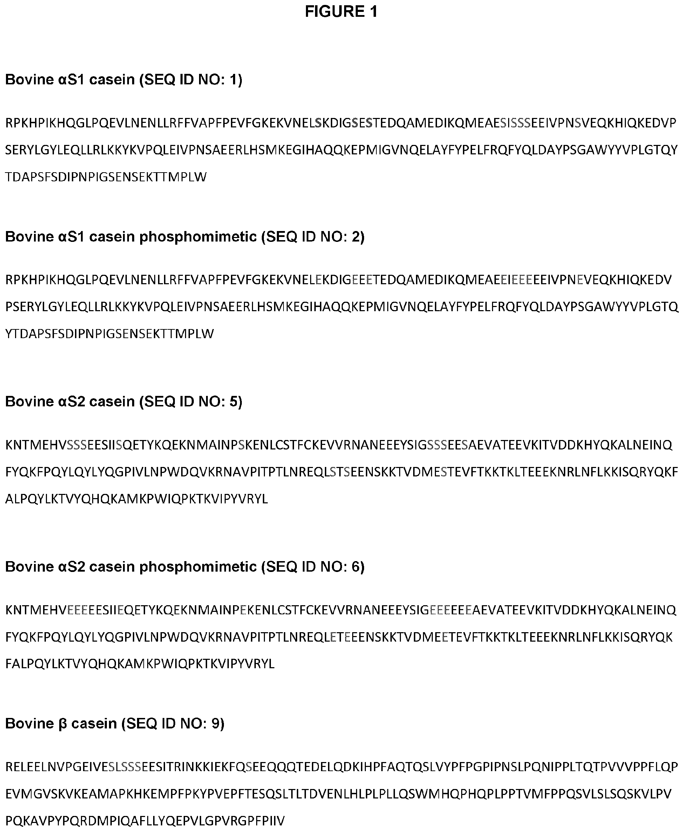

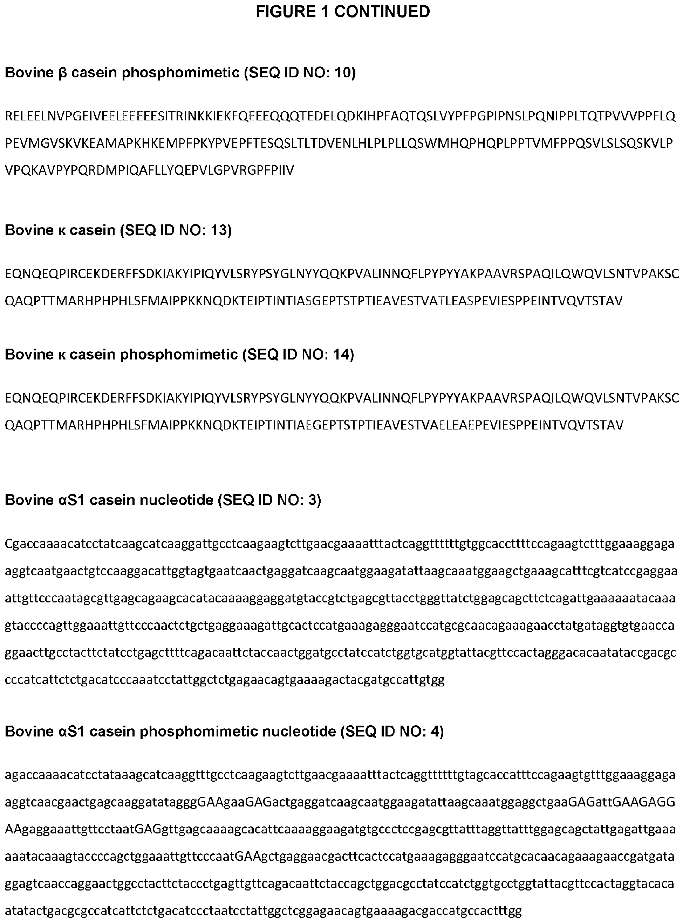

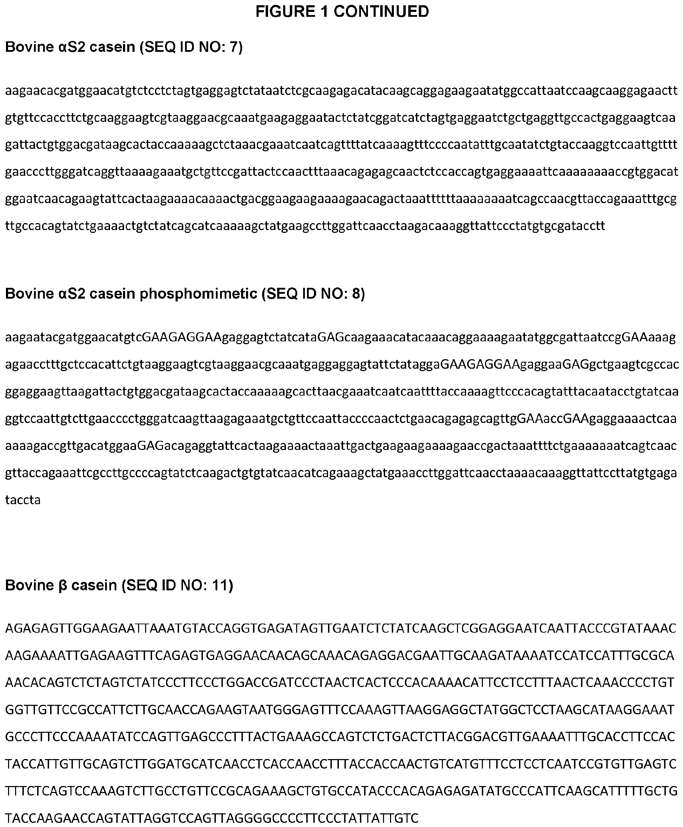

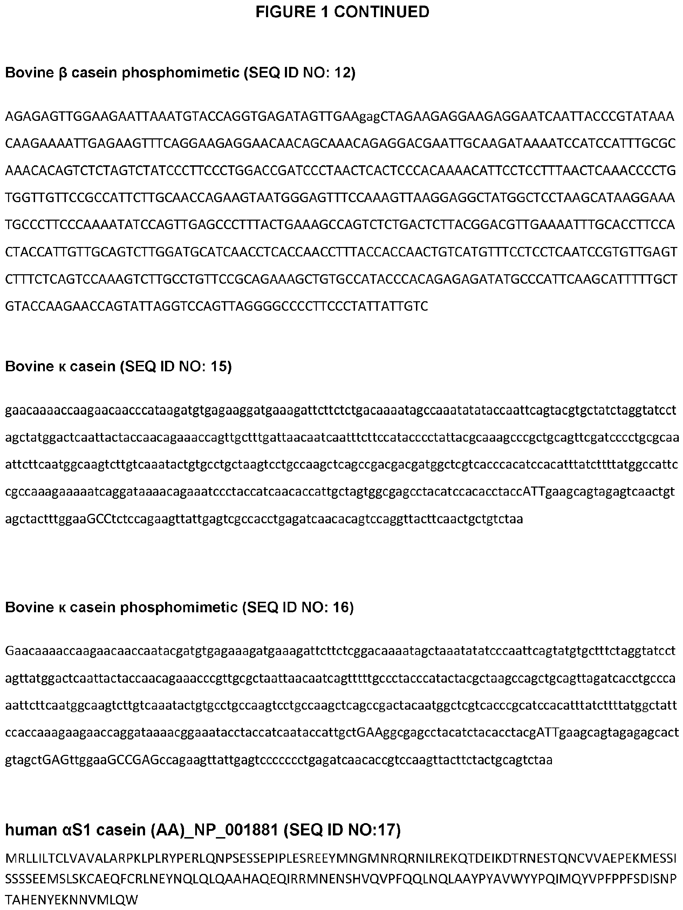

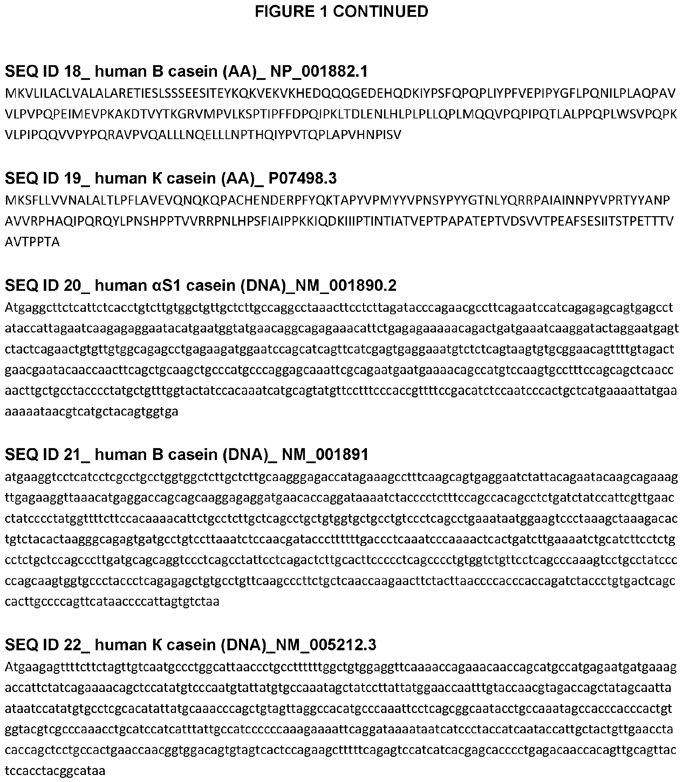

The casein protein encoded by the polynucleotide can be any mammalian casein protein, preferably bovine or human casein proteins at least 70% identical to casein αs1 having the amino acid sequence of SEQ ID: 1 or 17, casein αs2 having the amino acid sequence of SEQ ID: 5, casein β having the amino acid sequence of SEQ ID: 9 or 18 or casein κ having the amino acid sequence of SEQ ID:13 or 19. The polynucleotide can have the respective nucleotide sequence of SEQ ID NO: 4, 7, 11, 15, 20, 21, or 22 as shown in **Figure 1****.** The Sequence identity of other mammalian caseins to bovine casein can be seen below:

| | | **Casein** | | | |
|---|---|---|---|---|---|
| | **Species** | **aS1** | **beta** | **kappa** | **aS2** |
| **Sequence identity to bovine** | **buffalo** | 95% | 97% | 93% | 95% |
| | **goat** | 88% | 90% | 85% | 87% |
| | **sheep** | 88% | 91% | 85% | 89% |
| | **pig** | 47% | 67% | 54% | 62% |
| | **camel** | 45% | 69% | 58% | 58% |
| | **donkey** | 43% | 61% | 57% | 60% |
| | **dog** | 40% | 61% | 57% | 41% |
| | **human** | 32% | 56% | 53% | no as2 |
| | **horse** | ND | 59% | 57% | ND |

In one aspect the recombinant host cell or organism is genetically engineered to co-express casein κ having the amino acid sequence at least 70% identical to SEQ ID: 13 or 19 in combination with casein αs1 having the amino acid sequence at least 70% identical to SEQ ID: 1 or 17, casein αs2 having the amino acid sequence at least 70% identical to SEQ ID: 4 and/or casein β having the amino acid sequence at least 70% identical to SEQ ID: 9 or 21.

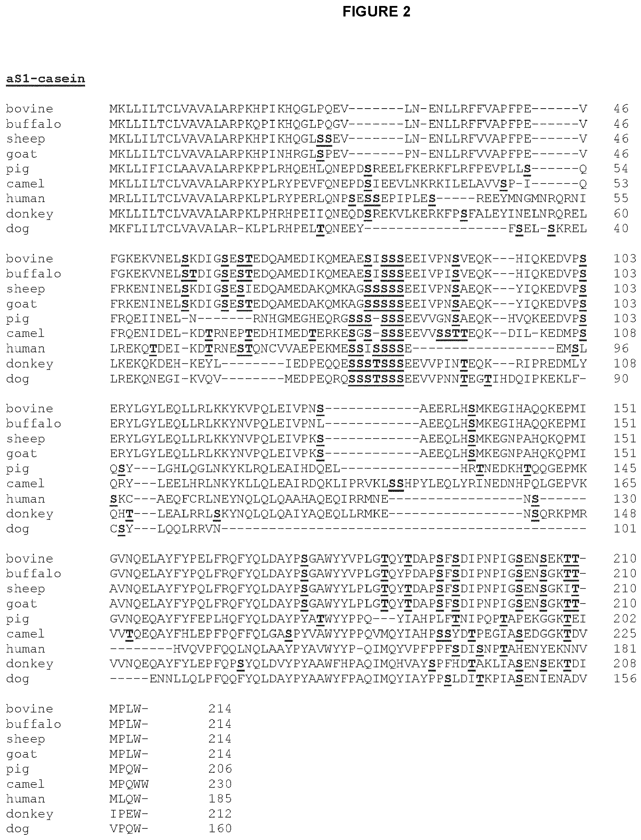

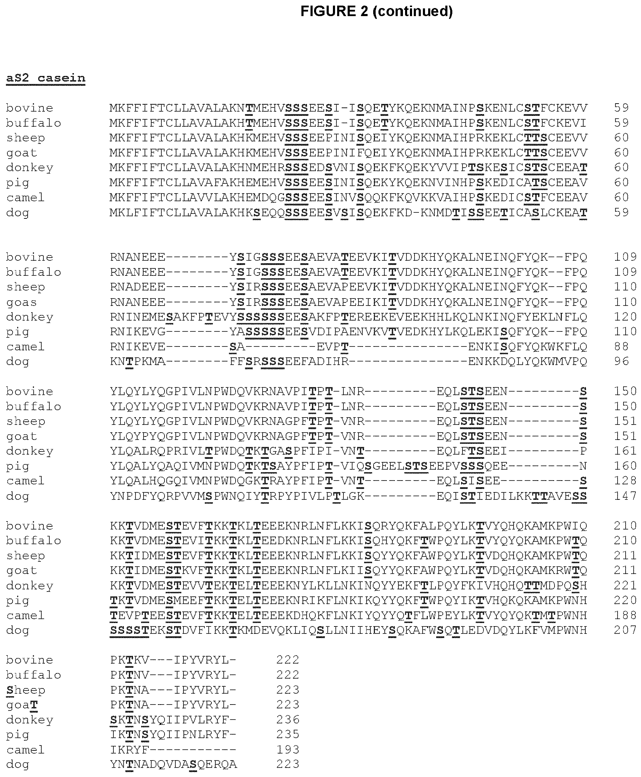

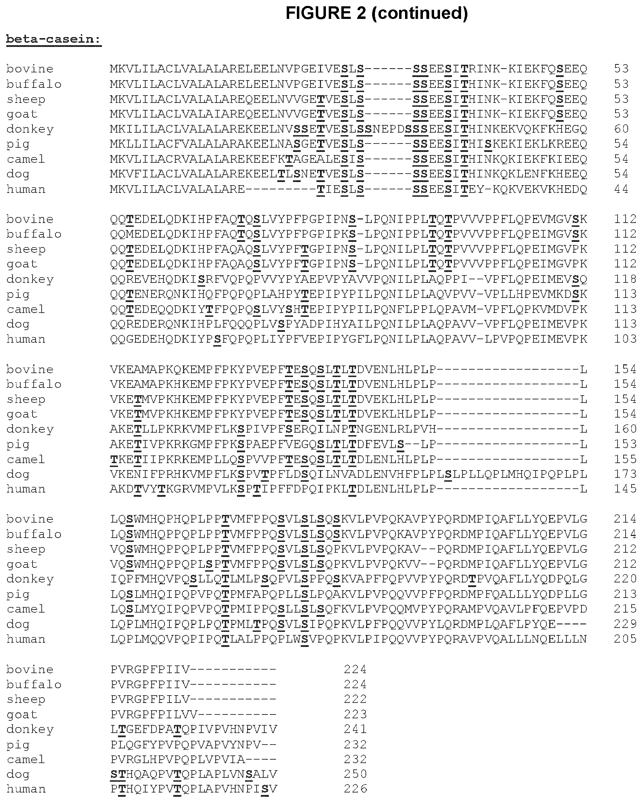

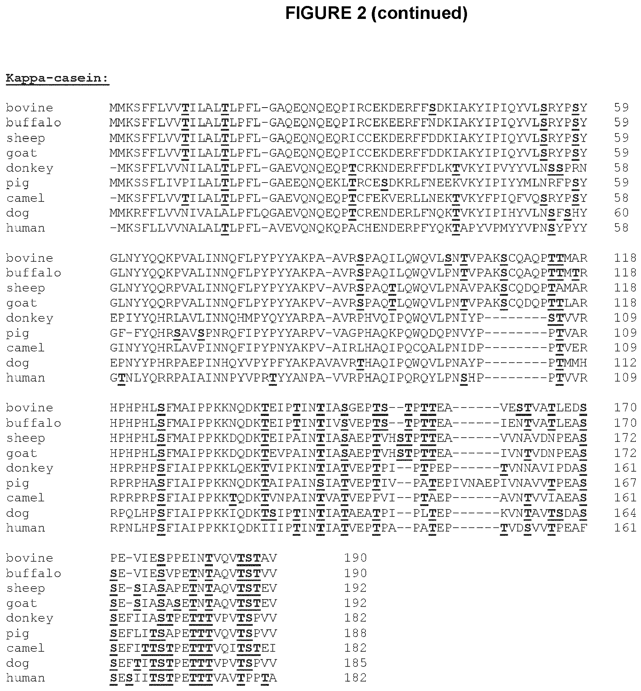

Optionally, the polynucleotide encoding the mammalian casein protein can be modified wherein one or more nucleotides encoding serine or threonine of the casein have been replaced with nucleotides encoding glutamic acid or asparagine. Such casein proteins are called phosphomimetic casein proteins, and the positions underlined in the alignments of Figure 2 would be eligible for such modifications. For these proteins, a co-expression with an hFAM20c kinase such as that shown in SEQ ID NO:23 is not necessary. Accordingly, the polynucleotide encoding the casein protein can be 70% identical to a polynucleotide having a sequence selected from the group consisting of SEQ ID NO: 2, 6, 10 or 14 encoded by a polynucleotide at least 70% identical to SEQ ID NO: 4, 8, 12 or 16.

In the context of the invention, the recombinant host cell or organism can be a bacterium, a yeast cell or a filamentous fungus. Preferred bacteria are *Escherichia coli* and *Bacillus subtilis,* preferred fungi are *Trichoderma reesei, Aspergillus oryzae* and *Aspergillus niger* and preferred yeast cells are selected from the group of Kluyveromyces sp., Pichia sp., Saccharomyces sp., Tetrahymena sp., Yarrowia sp., Hansenula sp., Blastobotrys sp., Candida sp., Zygosaccharomyces sp., and Debaryomyces sp, most preferably wherein the host cell is *Kluyveromyces lactis* or *Pichia pastoris.*

In some embodiments, the heterologous secretion signal introduced into the recombinant host cell or organism is not a casein secretion signal. Preferably, the heterologous secretion signal is selected from the group consisting of pOst1-aMF having the SEQ ID: 24, aMF having the SEQ ID NO: 25 or 26, or Killer protein having SEQ ID NO: 27.

When the host cell is *Kluyveromyces lactis,* the preferred promoter is a constitutive or an inducible promoter selected from the group consisting of: SEQ ID NO: 27, PDC1, KIADH3, *T.Reesei* CBH1 or Lac4. When the host cell is P. pastoris, the promoter can be a constitutive or an inducible promoter selected from the group consisting of Aox1, GAP, G1, DAS, FLD1, LRA3, THI11, TEF1 or GCW14.

In one specific aspect, the recombinant host cell is *Kluyveromyces lactis* or *Pichia pastoris,* the heterologous secretion signal is pOst1-aMF having the SEQ ID: 24; and the host cell is genetically engineered to co-express casein κ having the amino acid sequence at least 70% identical to SEQ ID: 13, 14, or 19 in combination with casein αs1 having the amino acid sequence at least 70% identical to SEQ ID: 1, 2 or 17, casein αs2 having the amino acid sequence at least 70% identical to SEQ ID: 5 or 6 and/or casein β having the amino acid sequence at least 70% identical to SEQ ID:9, 10 or 18.

In cases where the casein is not a phosphomimetic, for example where the casein has an amino acid sequence at least 70% identical to SEQ ID NO: 1, 4, 8, 12 or 17-19, at least one casein protein may be co-expressed with a kinase such as a bovine or human casein kinase I or II, or FAM20c kinase having at least 70% sequence identity to the SEQ ID: 23.

The invention also relates to the use of the recombinant host organism described above for manufacturing at least one casein protein of interest as well to method of producing casein proteins of interest.

Specifically, the invention relates to a new method of producing at least one casein protein of interest, comprising the steps of:
a. Expressing a polynucleotide encoding at least one casein protein having an amino acid sequence 70% identical to SEQ ID NO: 1, 2, 5, 6, 9, 10, 13 or 14 linked to a secretion signal in a recombinant host organism according to any one of claims 1 to 15; and
b. Culturing the host organism in a culture medium under conditions sufficient to allow for secretion of at least one casein protein into the culture medium.

In one aspect, the casein protein in step (a) is co-expressed with at least a second different casein protein having an amino acid sequence at least 70% identical to any one of SEQ ID NO: 1, 2, 5, 6, 9, 10, 13 or 14 and/or co-expressed with a FAM20c kinase having an amino acid sequence at least 70% identical to SEQ ID: 23.

The invention also relates to a casein protein produced by the method above and specifically to a casein protein having the amino acid sequence of SEQ ID: 2, 6, 10 or 14. Also encompassed is a micelle comprising such a casein and a composition, such as a food product, comprising such a casein or micelle.

Furthermore, the invention provides a method of increasing the secretion of casein from a recombinant *Kluyveromyces lactis* or *Pichia pastoris* host cell, comprising:
(i) Integrating a polynucleotide encoding at least two casein proteins having at least 70% amino acid identity to SEQ ID NO: 1, 2, 5, 6, 9, 10, 13 or 14 and one Fam20c kinase having at least 70% amino acid identity to SEQ ID NO: 23 into the host cell genome, wherein the polynucleotide is linked to the heterologous secretion signal pOst1-aMF having the SEQ ID: 24 and to a promoter having SEQ ID NO:27, and wherein the polynucleotide encodes casein κ having an amino acid sequence 70% identical to SEQ ID: 13, 14, or 19 in combination with casein αs1 having the amino acid sequence at least 70% identical to SEQ ID: 1, 2 or 17, casein αs2 having the amino acid sequence at least 70% identical to SEQ ID: 5 or 6 and/or casein β having the amino acid sequence at least 70% identical to SEQ ID: 9, 10 or 18,
(ii) Optionally Co-expressing at least two casein proteins and one Fam20c kinase having an amino acid sequence at least 70% identical to SEQ ID: 23 in the host cell, and
(iii) Culturing the host cell in a culture medium allowing for secretion of the casein proteins.

In one specific aspect, the method of increasing the secretion of casein from a recombinant *Kluyveromyces lactis* or *Pichia pastoris* host cell, comprises:
a. Integrating a polynucleotide encoding at least two casein proteins into the host cell genome, wherein the polynucleotide is linked to the heterologous secretion signal pOst1-aMF having the SEQ ID: A and to a promoter, and wherein the polynucleotide encodes casein κ having an amino acid sequence 70% identical to SEQ ID: 14 in combination with casein αs1 having the amino acid sequence at least 70% identical to SEQ ID: 2, casein αs2 having the amino acid sequence at least 70% identical to SEQ ID: 6 and/or casein β having the amino acid sequence at least 70% identical to SEQ ID: 10.
b. Co-expressing at least two casein proteins in the host cell, and
c. Culturing the host cell in a culture medium allowing for secretion of the casein proteins.

### DETAILED DESCRIPTION

As mentioned above, the present invention provides new recombinant cells and organisms and methods for the expression of caseins using several different approaches individually or in combination. Specifically, one approach is applying specific heterologous secretion signals or promoters to enhance yield and/or secretion. A second option is the provision of phosphomimetic caseins with changes in their amino acid composition, such as SEQ ID NO: 2, 5, 10 and 14. A third approach is the co-expression of different caseins, such as casein κ with one or several different caseins. These approaches have several advantages. First, the use of heterologous secretion signals that are not wildtype casein secretion signals improves secretion. Second, changes of the amino acid composition of some of the caseins avoid the phosphorylation step and therefore the need of co-expressing a kinase, such as FAM20c having SEQ ID NO: 23. Finally, the use of a combinatorial approach to express the caseins boosts their yield and promotes their correct folding. It is possible that the co-expression of the proteins promotes a scenario where one protein functions as a chaperone to the other, therefore leading to correct folding and casein micelles.

The term "host organism" in the context of the application is an organism selected from the group consisting of bacteria, yeast and filamentous fungi. Preferred bacteria are *Escherichia coli* and *Bacillus subtilis,* preferred fungi are *Trichoderma reesei, Aspergillus oryzae* and *Aspergillus niger* and preferred yeast cells are selected from the group of Kluyveromyces sp., Pichia sp., Saccharomyces sp., Tetrahymena sp., Yarrowia sp., Hansenula sp., Blastobotrys sp., Candida sp., Zygosaccharomyces sp., and Debaryomyces sp, most preferably wherein the host organism is *Kluyveromyces lactis* or *Pichia pastoris.*

The term "host cell" can include the host organisms above as well as cells from cell culture, such as bovine mammary epithelial cells (Sakamoto et al., 2005, Monzani et al., 2011).

The term "genetically engineered" as used herein relates to an organism that includes exogeneous, non-native nucleic acid sequences either maintained episomally in an expression vector or integrated into the genome of the host organism.

In the context of the invention, a "casein protein" refers to a protein at least 30%, at least 40%, at least 50%, at least 60%, at least 70%, at least 80%, at least 90%, at least 95% or completely identical to a natural mammalian casein protein. Preferred casein proteins are bovine or human casein proteins. In the context of the invention, preferred natural casein proteins are those having at least 70% identity to SEQ ID NO:1 (bovine αS1), SEQ ID NO: 5 (bovine αS2), SEQ ID NO: 9 (bovine β), SEQ ID NO:13 (bovine κ), SEQ ID NO: 17 (human αS1), SEQ ID NO: 18 (human β), or SEQ ID NO:19 (human κ), or any of the casein proteins shown in Figures 1 and 2.

"Sequence identity" or "% identity" refers to the percentage of residue matches between at least two polypeptide sequences aligned using a standardized algorithm. Such an algorithm may insert, in a standardized and reproducible way, gaps in the sequences being compared in order to optimize alignment between two sequences, and therefore achieve a more meaningful comparison of the two sequences. For purposes of the present invention, the sequence identity between two amino acid sequences is determined using the NCBI BLAST program version 2.3.0 (Jan-13-2016) (Altschul et al., Nucleic Acids Res. (1997) 25:3389-3402). Sequence identity of two amino acid sequences can be determined with blastp set at the following parameters: Matrix: BLOSUM62, Word Size: 3; Expect value: 10; Gap cost: Existence = 11, Extension = 1; Compositional adjustments: Conditional compositional score matrix adjustment. As can be seen from Figure 2, casein proteins from different species are highly conserved, so that an identity of 70% based on bovine (SEQ ID NO: 1, 4, 8, 12) or human (SEQ ID NO: 20-22) caseins includes other mammalian casein proteins.

A "polynucleotide" refers to a molecule comprising a polymer of nucleotide monomers covalently bonded in a chain. In the context of the invention, the polynucleotide is DNA (deoxyribonucleic acid) chain. Said term is not meant herein to refer to a specific length of the molecule and is therefore herein interchangeably used with the term "DNA". In the context of the present invention, the polynucleotides encoding the caseins can be codon optimized for the host organism. For example, the nucleotide sequences shown as SEQ ID NO: 3, 4, 7, 8, 11, 12, 15 and 16 are codon optimized for expression in yeast host cells, specifically for *Pichia pastoris.*

A "polypeptide" refers to a molecule comprising a polymer of amino acids linked together by peptide bonds. Said term is not meant herein to refer to a specific length of the molecule and is therefore herein interchangeably used with the term "protein". When used herein, the term "polypeptide" or "protein" also includes a "polypeptide of interest" or "protein of interest" which is expressed by the expression cassettes or vectors or can be isolated from the host cells of the invention. A polypeptide comprises an amino acid sequence, and, thus, sometimes a polypeptide comprising an amino acid sequence is referred to herein as a "polypeptide comprising a polypeptide sequence". Thus, herein the term "polypeptide sequence" is interchangeably used with the term "amino acid sequence". The term "amino acid" or "aa" refers to naturally occurring amino acids. Naturally occurring amino acids are those encoded by the genetic code.

The terms "integrated into the host genome" and "maintained episomally" refer to known methods of including polynucleotides into host cells. In the context of the present invention, the polynucleotide encoding the casein protein is linked to a heterologous secretion signal.

The "heterologous secretion signal" (used interchangeably with "signal sequence", "leader sequence" or "signal peptide") of the invention is not a native secretion signal included in the host organism genome. In preferred embodiments, the secretion signal is not a native casein secretion signal but is selected from a pOst1-AMF secretion signal having SEQ ID NO: 24 (from J. J. Barrero, J. C. Casler, F. Valero, P. Ferrer, and B. S. Glick, "An improved secretion signal enhances the secretion of model proteins from Pichia pastoris," Microb Cell Fact, vol. 17, no. 1, p. 161, Oct. 2018, doi: 10.1186/s12934-018-1009-5*.)*, an AMF secretion signal having SEQ ID NO:25 or 26 and a Killer secretion signal having SEQ ID NO:27 shown below.

Secretion signals (SS):
SEQ ID NO: 24 pOst1-AMF 
ATGAGGCAGGTTTGGTTCTCTTGGATTGTGGGATTGTTCCTATGTTTTTTCAACGTGTCTTCTGCTGCTCCAGTCAACACTAC
AACAGAAGATGAAACGGCACAAATTCCGGCTGAAGCTGTCATCGGTTACTCAGATTTAGAAGGGGATTTCGATGTTGCTGT
TTTGCCATTTTCCAACAGCACAAATAACGGGTTATTGTTTATAAATACTACTATTGCCAGCATTgctgCTAAAGAAGAAGGGG
TATCTTTGGATAAAAGAGAGGCTGAAGCT
SEQ ID 25_aMF K. Lactis SS (DNA) 
ATGAAATTCTCTACTATATTAGCCGCATCTACTGCTTTAATTTCCGTTGTTATGGCTGCTCCAGTTTCTACCGAAACTGACATC
GACGATCTTCCAATATCGGTTCCAGAAGAAGCCTTGATTGGATTCATTGACTTAACCGGGGATGAAGTTTCCTTGTTGCCTG
TTAATAACGGAACCCACACTGGTATTCTATTCTTAAACACCACCATCGCTGAAGCTGCTTTCGCTGACAAGGATGATCTCGA
GAAAAGAGAGGCTGAAGCT
SEQ ID 26_aMF pre-sequence P. Pastoris SS (DNA)
   ATGAGATTTCCTTCAATTTTTACTGCTGTTTTATTCGCAGCATCCTCCGCATTAGCT
SEQ ID 27_Killer protein S. Cerevisiae SS (DNA)
   ATGACTAAGCCAACCCAAGTATTAGTTAGATCCGTCAGTATATTATTTTTCATCACATTACTACATCTAGTCGTAGCT
SEQ ID 31_α-amylase signal sequence
   ATGGTCGCTTGGTGGTCTTTGTTTCTGTACGGTCTTCAGGTCGCTGCACCTGCTTTGGCT
SEQ ID 32_Glucoamylase signal sequence
   ATGTCTTTTAGATCCTTGTTGGCTTTGTCTGGTTTGGTTTGTTCTGGTTTGGCT
SEQ ID 33_Serum albumin signal sequence
   ATGAAGTGGGTTACCTTTATCTCTTTGTTGTTTCTTTTCTCTTCTGCTTACTCT
SEQ ID 34 Inulinase presequence
   ATGAAGTTAGCATACTCCTTGTTGCTTCCATTGGCAGGAGTCAGTGCT
SEQ ID 35 Invertase signal sequence
   ATGCTTTTGCAAGCTTTCCTTTTCCTTTTGGCTGGTTTTGCAGCCAAAATATCTGCA
SEQ ID NO 36 Lysozyme signal sequence
   ATGCTGGGTAAGAACGACCCAATGTGTCTTGTTTTGGTCTTGTTGGGATTGACTGCTTTGTTGGGTATCTGTCAAGGT

In the context of the invention, the promoter can be any promoter that allows for translation of the polynucleotide in the host organism. When the host cell is *Kluyveromyces lactis,* the preferred promoter is a constitutive or an inducible promoter selected from the group consisting of: SEQ ID NO: 27, PDC1, KIADH3, *T.Reesei* CBH1 or Lac4. When the host cell is P. pastoris, the promoter can be a constitutive or an inducible promoter selected from the group consisting of Aox1, GAB, G1, DAS, FLD1, LRA3, THI11, TEF1 or GCW14.

For *Pichia pastoris,* a suitable promoter is ADH3, pGAP, G1 or most preferably pAOX1 having the sequence shown below:
**>SEQ ID NO:37** pAOX1

GATCTAACATCCAAAGACGAAAGGTTGAATGAAACCTTTTTGCCATCCGACATCCACAGGTCCATTCTCACACATAAGTGCC
AAACGCAACAGGAGGGGATACACTAGCAGCAGACCGTTGCAAACGCAGGACCTCCACTCCTCTTCTCCTCAACACCCACTTT
TGCCATCGAAAAACCAGCCCAGTTATTGGGCTTGATTGGAGCTCGCTCATTCCAATTCCTTCTATTAGGCTACTAACACCATG
ACTTTATTAGCCTGTCTATCCTGGCCCCCCTGGCGAGGTTCATGTTTGTTTATTTCCGAATGCAACAAGCTCCGCATTACACC
CGAACATCACTCCAGATGAGGGCTTTCTGAGTGTGGGGTCAAATAGTTTCATGTTCCCCAAATGGCCCAAAACTGACAGTTT
AAACGCTGTCTTGGAACCTAATATGACAAAAGCGTGATCTCATCCAAGATGAACTAAGTTTGGTTCGTTGAAATGCTAACGG
CCAGTTGGTCAAAAAGAAACTTCCAAAAGTCGGCATACCGTTTGTCTTGTTTGGTATTGATTGACGAATGCTCAAAAATAAT
CTCATTAATGCTTAGCGCAGTCTCTCTATCGCTTCTGAACCCCGGTGCACCTGTGCCGAAACGCAAATGGGGAAACACCCGC
TTTTTGGATGATTATGCATTGTCTCCACATTGTATGCTTCCAAGATTCTGGTGGGAATACTGCTGATAGCCTAACGTTCATGA
TCAAAATTTAACTGTTCTAACCCCTACTTGACAGCAATATATAAACAGAAGGAAGCTGCCCTGTCTTAAACCTTTTTTTTTATC
ATCATTATTAGCTTACTTTCATAATTGCGACTGGTTCCAATTGACAAGCTTTTGATTTTAACGACTTTTAACGACAACTTGAGA
AGATCAAAAAACAACTAATTATTCGAAACG

For *K. lactis,* a suitable promoter is p350, pLAC4 or PDC1, most preferably p350 having the sequence below:
**SEQ ID 28_ p350**

GTCAGTACCGACTGGGATCTTGGCAGCTTGAACGGCATCAGCAGTAGCGTATTGTTGGAAAGAACCGTCGTGAGTGTAACC
GGACAAGTCAGCTTCTGGACAGTTGGATTCGTTGGACAATTCACAGTATTCACAGGACATACAAGAACCGTTCAACCATTTG
ATACCAGCGAAGTCACCAATCTTCCAGCCCTTGACGTTTTCACCCATAGCAACAACGACACCAGCACCTTCGTGACCACCAA
CTAATGGCAATTTGGTTGGCAAAGGCCAGTCACCCTTCCATGCGTGCAAATCGGTGTGACAGACACCGGAGTACTTGACGT
TGATCAAAAGTTCGTTAGCCTTTGGCTTTGGAACTGGGATGTCCTTGTATTGCAATTCACCACCGTTTTCGTAGAAGATAACA
CCCTTTTGAGTTTCTGGGATAGATGCGGCCATTTTATCTTTTTTTAGTATAGAGTTTGTGTGTTTAAAGCTTGTTTATGTTTCA
ATTGAAACTATTGTTTTTGCTTGATGATGATGTAGGGCAAAACAGAAACAACATTGTACAAATGGATATAGAGAGACCTACT
ACAAAAGTGGATGGATTGTCAGAATCGACCGGGTGTTTATATACATATTCCAGAAGACCAAATGGCTGGTCTAATTGTATA
CAAGTAAGACAGCTGATTTCTAGCTGGTGTGAAAATAGCACACTTTGAGAAAAATACATACCACCCTGCAACAGGTGTTAA
CCGTTTAAGACCTAGAAATAACCGCAGTATATAAAGATCAATCGAATACTTGGGTTGGTGAAAAGCAACCCAAAATACATC
ATCTGCTCATATGATTGTGATCTGATTGTGAATAGATCAATACACTACTAGAATAGAAGAACATCACACGCTATTTTTTTTTC
CTCTTCTGTCCTACTCGGAGAAACAGAAAGTATCAGGTCACCACCGGACATGTCATACTATCTAGATAATCGGGTATGATTA
ACAGCGACATAGCTATGTGATTATCTGCCCCATTACTTAAAGTAAGGTTTCACTTTCGGACTAAGGACGAATGACACAAGAC
ACAGAACGCTCACACGGTTGAAACATTTTTTTTCCTGATCCTTTTGACCCTGTTTGTTGTCCCTCTTTTACGATGGGAGAATTG
AATCATCCGCCATTAAACCGAATGTGATAGTTTTTCCAGTTTTTCTTCCCCTCCCTACATCCATTTCTAAAGAATTCACGGACA
TTTTCGCATGAATGGGGAAAGTTGACTCAGTTGACTCTATTTGGTTCCTCAGCCTCGACTGCTTTGCTTCATCTTCTCTATGG
GCCAATGAGCTAATGAGCACAATGTGCTGCGAAATAAAGGGATATCTAATTTATATTATTACATTATAATATGTACTAGTGT
GGTTATTGGTAATTGTACTTAATTTTGATATATAAAGGGTGGATCTTTTTCATTTTGAATCAGAATTGGAATTGCAACTTGTC
TCTTGTCACTATTACTTAATAGTAATTATATTTCTTATTAACCTTTTTTTTAAGTCAAAACACCAAGGACAAGAACTACTCTTCA
AAGGTATTTCAAGTTATCATACGTCTCACACACGCTTCACAGTTTCAAGTAAAAAAAAAGAATATTACACA

The terminator can be any terminator known to end translation in the host organism. For *Pichia pastoris,* a suitable terminator is CYC1tt or AOX1tt. For *K. lactis,* a suitable terminator is pLAC4tt.

In the context of the invention, the host organism can be genetically engineered to co-express 2, 3, or 4 different casein proteins. These casein proteins can be natural casein proteins from the same organism or can be from different organisms. In addition, the casein proteins can be phosphomimetics, where several serine (S) or threonine (T) residues have been replaced with glutamic acid (E) or asparagine (N). For example, the casein proteins can have the amino acid sequence shown as SEQ ID NO: 2, 6, 10 or 14, encoded by the nucleotide sequence having the respective SEQ ID NO: 4, 8, 12 or 16.

It is particularly preferred to combine a polynucleotide encoding a κ casein such as SEQ ID NO: 13, 14 or 19 with a polynucleotide encoding an α or β casein. Preferred constructs can be seen in Tables 1 and 2 below.

In addition, when the casein proteins are not phosphomimetics, the casein proteins can be co-expressed with a kinase, such as hFAM20c having an amino acid sequence at least 70% identical to SEQ ID NO:23 and capable of phosphorylating the casein proteins.

The term "co-expression" in the context of the invention means that at least two polynucleotides are expressed using at least one, two or more expression cassettes. In some cases, at least two polynucleotides are on one expression cassette with at least one open reading frame.

**Table 1 K. lactis constructs**

| K. lactis | Cassette 1 | | | Cassette 2 | | | Cassette 3 | | | Terminator |
|---|---|---|---|---|---|---|---|---|---|---|
| Combination no. | promoter | SS | ORF | promoter | SS | ORF | promoter | SS | ORF | |
| 1 | pLAC4 | aMF | aS1_m | pLAC4 | -- | -- | pLAC4 | -- | -- | LAC4 tt |
| 2 | | aMF | K_m | | -- | -- | | -- | -- | |
| 3 | | aMF | aS1_m | | aMF | K_m | | -- | -- | |
| 4 | | pOstl- aMF | K_m | | -- | -- | | -- | -- | |
| 5 | | aMF | aS1_m | | pOstl- aMF | K_m | | -- | -- | |
| 6 | | aMF | aS1 | | -- | hFAM20c | | -- | -- | |
| 7 | | aMF | K | | -- | hFAM20c | | -- | -- | |
| 8 | | pOstl- aMF | K | | -- | hFAM20c | | -- | -- | |
| 9 | | aMF | aS1 | | aMF | K | | -- | hFAM20c | |
| 10 | | aMF | aS1 | | pOstl- aMF | K | | -- | hFAM20c | |

**Table 2 P.pastoris constructs**

| P. pastoris | AOX1 locus | | | | | | | | | | GAP locus | | |
|---|---|---|---|---|---|---|---|---|---|---|---|---|---|
| | Cassette 1 | | | Cassette 2 | | | Cassette 3 | | | Termin ator | promo ter | ORF | Termi nator |
| Combi nation no. | promote r | SS | ORF | 2A | SS | ORF | 2A | SS | ORF | CYC1 tt/AOX 1 tt | | | |
| 1 | pAOX1/ pGAP | aMF | aS1_ m | | | | | | | | | | |
| 2 | | pOstl- aMF | K_m | | | | | | | | | | |
| 3 | | aMF | aS1_ m | T2A_ 1 | aMF | K_m | | | | | | | |
| 4 | | pOstl- aMF | K_m | | | | | | | | | | |
| 5 | | aMF | aS1_ m | T2A_ 1 | pOstl -aMF | K_m | | | | | | | |
| 6 | | aMF | aS1 | | | | | | | | pGAP | hFA M20c | AOX 1 TT |
| 7 | | aMF | K | | | | | | | | | | |
| 8 | | aMF | aS1 | T2A_ 1 | | | | | | | | | |
| 9 | | pOstl- aMF | K | | aMF | K | | | | | | | |
| 10 | | aMF | aS1 | T2A_ 1 | pOstl -aMF | K | | | | | | | |
| 11 | | a-amylase | aS1 | T2A_ 1 | aMF | beta | T2A _2 | pOst1 -aMF | K | | | | |
| 12 | | Killer protein | | | | | | | | | | | |
| 13 | | HSA | | | | | | | | | | | |

In the examples of the invention, the host organism is either *Kluyveromyces lactis* or *Pichia pastoris* as described in the Examples.

### Use of the Recombinant host organism and method of producing casein proteins

The present invention also pertains to the use of the recombinant host cell or organism for producing at least one casein protein of interest.

Specifically, the invention relates to a new method of producing at least one casein protein of interest, comprising the steps of:
c. Expressing a polynucleotide encoding at least one casein protein having an amino acid sequence 70% identical to SEQ ID NO: 1, 2, 5, 6, 9, 10, 13 or 14 linked to a secretion signal in a recombinant host organism according to any one of claims 1 to 15; and
d. Culturing the host cell or organism in a culture medium under conditions sufficient to allow for secretion of the at least one casein protein into the culture medium.

In one aspect, the casein protein in step (a) is co-expressed with at least a second different casein protein having an amino acid sequence at least 70% identical to any one of SEQ ID NO: 1, 2, 5, 6, 9, 10, 13 or 14 and/or co-expressed with a FAM20c kinase having an amino acid sequence at least 70% identical to SEQ ID: 23.

The invention also relates to a casein protein produced by the method above and specifically to a casein protein having the amino acid sequence of SEQ ID: 2, 6, 10 or 14. Also encompassed is a micelle comprising such a casein and a composition, such as a food product, comprising such a casein or micelle.

Furthermore, the invention provides a method of increasing the secretion of casein from a recombinant *Kluyveromyces lactis* or *Pichia pastoris* host cell, comprising:
(iv) Integrating a polynucleotide encoding at least two casein proteins having at least 70% amino acid identity to SEQ ID NO: 1, 2, 5, 6, 9, 10, 13 or 14 and one Fam20c kinase having at least 70% amino acid identity to SEQ ID NO: 23 into the host cell genome, wherein the polynucleotide is linked to the heterologous secretion signal pOst1-aMF having the SEQ ID: 24 and to a promoter having SEQ ID NO:27, and wherein the polynucleotide encodes casein κ having an amino acid sequence 70% identical to SEQ ID: 13, 14, or 19 in combination with casein αs1 having the amino acid sequence at least 70% identical to SEQ ID: 1, 2 or 17, casein αs2 having the amino acid sequence at least 70% identical to SEQ ID: 5 or 6 and/or casein β having the amino acid sequence at least 70% identical to SEQ ID: 9, 10 or 18,
(v) Optionally co-expressing at least two casein proteins and one Fam20c kinase having an amino acid sequence at least 70% identical to SEQ ID: 23 in the host cell, and
(vi) Culturing the host cell in a culture medium allowing for secretion of the casein proteins.

In one specific aspect, the method of increasing the secretion of casein from a recombinant *Kluyveromyces lactis* or *Pichia pastoris* host cell, comprises:
a. Integrating a polynucleotide encoding at least two casein proteins into the host cell genome, wherein the polynucleotide is linked to the heterologous secretion signal pOst1-aMF having the SEQ ID: 24 and to a promoter, and wherein the polynucleotide encodes casein κ having an amino acid sequence 70% identical to SEQ ID: 14 in combination with casein αs1 having the amino acid sequence at least 70% identical to SEQ ID: 2, casein αs2 having the amino acid sequence at least 70% identical to SEQ ID: 6 and/or casein β having the amino acid sequence at least 70% identical to SEQ ID: 10.
b. Co-expressing at least two casein proteins in the host cell, and
c. Culturing the host cell in a culture medium allowing for secretion of the casein proteins.

The examples describe the methods of the invention for *Kluyveromyces lactis* or *Pichia pastoris.* Known methods can be used for other host cells, such as *E.coli* or filamentous fungi.

It must be noted that as used herein, the singular forms "a", "an", and "the", include plural references unless the context clearly indicates otherwise. Thus, for example, reference to "an expression cassette" includes one or more of the expression cassettes disclosed herein and reference to "the method" includes reference to equivalent steps and methods known to those of ordinary skill in the art that could be modified or substituted for the methods described herein.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

The term "about" or "approximately" as used herein means within 20%, preferably within 10%, and more preferably within 5% of a given value or range. It includes also the concrete number, e.g., about 20 includes 20.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present invention are generally performed according to conventional methods well-known in the art. Generally, nomenclatures used in connection with techniques of biochemistry, enzymology, molecular and cellular biology, microbiology, genetics and protein and nucleic acid chemistry and hybridization described herein are those well-known and commonly used in the art.

The methods and techniques of the present invention are generally performed according to conventional methods well-known in the art and as described in various general and more specific references that are cited and discussed throughout the present specification unless otherwise indicated. See, e. g., Sambrook et al., Molecular Cloning: A Laboratory Manual, 3rd ed., Cold Spring Harbor Laboratory Press, Cold Spring Harbor, N. Y. (2001); Ausubel et al., Current Protocols in Molecular Biology, J, Greene Publishing Associates (1992, and Supplements to 2002); Handbook of Biochemistry: Section A Proteins, Vol I 1976 CRC Press; Handbook of Biochemistry: Section A Proteins, Vol II 1976 CRC Press. The nomenclatures used in connection with, and the laboratory procedures and techniques of, molecular and cellular biology, protein biochemistry, enzymology and medicinal and pharmaceutical chemistry described herein are those well-known and commonly used in the art.

### DESCRIPTON OF THE FIGURES

**Figure 1****:** Amino acid and nucleotide sequences of bovine casein proteins of the present invention
**Figure 2****:** Alignments of caseins from different species with possible phosphomimetic sites underlined

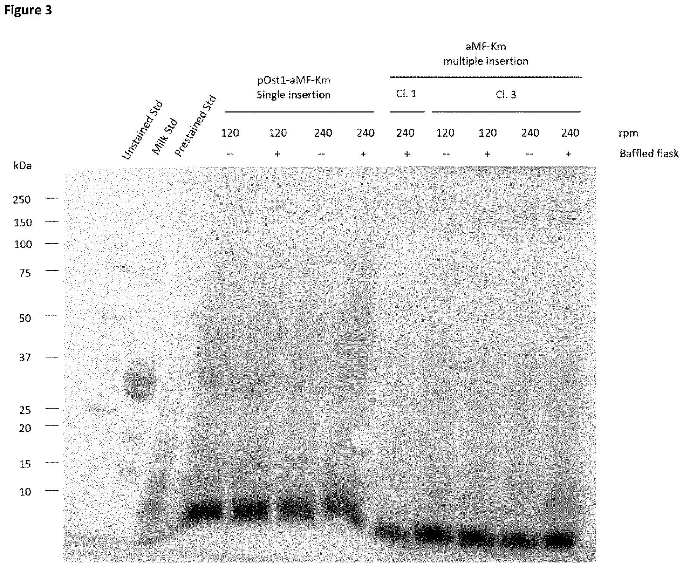

**Figure 3****:** SDS-PAGE analysis of K. lactis strains with genomic single copy insertion of pOst1-aMF-Kappa casein and multi-copy insertion (≥ 2) of aMF-Kappa casein.
**Figure 4****:** Western blot analysis of K. lactis strains with genomic single copy insertion of pOst1-aMF-Kappa casein and multi-copy insertion (≥ 2) of aMF-Kappa casein.

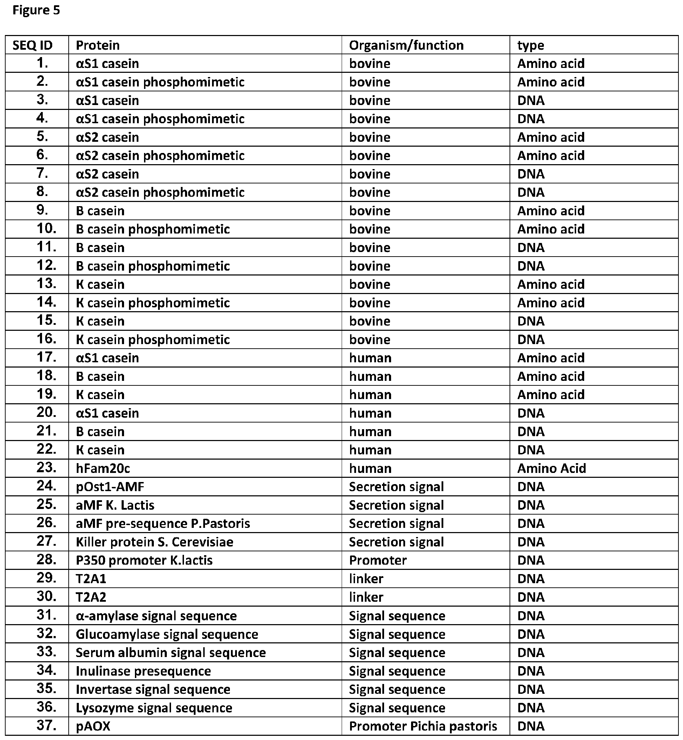

**Figure 5****:** Table of Sequences

### ITEMS

The following items further characterize the invention:
1. A recombinant host cell genetically engineered to express at least one casein protein, wherein the polynucleotide encoding the casein protein is integrated into the host cell genome or is maintained episomally and is linked to a heterologous secretion signal and to a promoter and is followed by a terminator sequence.
2. The recombinant host cell of item 1 wherein the host cell is genetically engineered to produce at least two, at least three or at least 4 different casein proteins.
3. The recombinant host cell of item 1 or 2, wherein the casein protein encoded by the polynucleotide is at least 70% identical to casein αs1 having the amino acid sequence of SEQ ID NO: 1, 2 or 17, casein αs2 having the amino acid sequence of SEQ ID NO: 5 or 6, casein β having the amino acid sequence of SEQ ID NO:9, 10 or 18 or casein κ having the amino acid sequence of SEQ ID NO: 13, 14 or 19.
4. The recombinant host cell of any one of items 1 to 3, wherein the host cell is genetically engineered to co-express casein κ having the amino acid sequence at least 70% identical to SEQ ID:13, 14 or 19 in combination with casein αs1 having the amino acid sequence at least 70% identical to SEQ ID: 1, 2 or 17, casein αs2 having the amino acid sequence at least 70% identical to SEQ ID: 5 or 6 and/or casein β having the amino acid sequence at least 70% identical to SEQ ID:9, 10 or 18.
5. The recombinant host cell of any one of items 1 to 4, wherein one or more the nucleotides encoding serine or threonine of the casein have been replaced with nucleotides encoding glutamic acid or aspartic acid.
6. The recombinant host cell of any one of items 1 to 5, wherein the polynucleotide encoding the casein has a nucleotide sequence selected from the group consisting of SEQ ID NO: 3, 4, 7, 8, 11, 12, 15, 16, or 20 to 22.
7. The recombinant host cell of any one of items 1 to 6, wherein the heterologous secretion signal is not a casein secretion signal.
8. The recombinant host cell of any one of the preceding items, wherein the host cell is an organism, such as a bacterium, a yeast or a filamentous fungus.
9. The recombinant host cell of item 8, wherein the organism is a bacterium preferably selected from the group consisting of *Escherichia coli* and *Bacillus subtilis,* a filamentous fungus selected from the group consisting of *Trichoderma reesei, Aspergillus oryzae* and *Aspergillus niger,* or a yeast cell preferably selected from the group of Kluyveromyces sp., Pichia sp., Saccharomyces sp., Tetrahymena sp., Yarrowia sp., Hansenula sp., Blastobotrys sp., Candida sp., Zygosaccharomyces sp., and Debaryomyces sp, preferably wherein the host cell is *Kluyveromyces lactis* or *Pichia pastoris.*
10. The recombinant host cell of item 9, wherein the host cell is a yeast cell and the heterologous secretion signal is selected from the group consisting of pOst1-aMF having the SEQ ID: 24, aMF having the SEQ ID NO: 25 or 26 or Killer protein having SEQ ID NO: 27.
11. The recombinant host cell of item 9 or 10, wherein the host cell is *Kluyveromyces lactis* or *Pichia pastoris,* preferably wherein in the promoter is SEQ ID NO: 28 or SEQ ID NO:37.
12. The recombinant host organism of any one of items 9 to 11, wherein:
   a. the host cell is *Kluyveromyces lactis* or *Pichia pastoris;*
   b. the heterologous secretion signal is pOst1-aMF having the SEQ ID: 24; and
   c. the host cell is genetically engineered to co-express casein κ having the amino acid sequence at least 70% identical to SEQ ID: 13, 14, or 19 in combination with casein αs1 having the amino acid sequence at least 70% identical to SEQ ID: 1, 2 or 17, casein αs2 having the amino acid sequence at least 70% identical to SEQ ID: 5 or 6 and/or casein β having the amino acid sequence at least 70% identical to SEQ ID:9, 10 or 18.
13. The recombinant host organism of any one of items 9 to 12, wherein at least one casein protein is co-expressed with a FAM20c kinase having at least 70% sequence identity to the SEQ ID: 23.
14. Use of a recombinant host cell of any one of items 1 to 13 for manufacturing at least one casein protein of interest.
15. A method of producing at least one casein protein of interest, comprising the steps of:
   a. Expressing a polynucleotide encoding at least one casein protein having an amino acid sequence 70% identical to SEQ ID NO: 1, 2, 5, 6, 9, 10, 13 or 14 linked to a secretion signal in a recombinant host cell according to any one of claims 1 to 15;
   b. Culturing the host cell in a culture medium under conditions sufficient to allow for secretion of the at least one casein protein into the culture medium.
16. The method of item 15, wherein the casein protein in step (a) is co-expressed with at least a second casein protein having an amino acid sequence at least 70% identical to any one of SEQ ID NO: 1, 2, 5, 6, 9, 10, 13 or 14 and/or co-expressed with a FAM20c kinase having an amino acid sequence at least 70% identical to SEQ ID: 23.
17. A casein protein produced by the method of item 15 or 16.
18. A casein protein having the amino acid sequence of SEQ ID: 2, 6, 10 or 14.
19. A micelle comprising one or several casein proteins according to item 17 or 18.
20. A composition comprising a casein protein of item 17 or 18 or a micelle of item 19.
21. A method of increasing the secretion of casein from a recombinant *Kluyveromyces lactis* or *Pichia pastoris* host cell, comprising:
   a. Integrating a polynucleotide encoding at least two casein proteins having at least 70% amino acid identity to SEQ ID NO: 1, 2, 5, 6, 9, 10, 13 or 14 and one Fam20c kinase having at least 70% amino acid identity to SEQ ID NO: 23 into the host cell genome, wherein the polynucleotide is linked to the heterologous secretion signal pOst1-aMF having the SEQ ID: 24 and to a promoter having SEQ ID NO:28 or 37, and wherein the polynucleotide encodes casein κ having an amino acid sequence 70% identical to SEQ ID: 13, 14, or 19 in combination with casein αs1 having the amino acid sequence at least 70% identical to SEQ ID: 1, 2 or 17, casein αs2 having the amino acid sequence at least 70% identical to SEQ ID: 5 or 6 and/or casein β having the amino acid sequence at least 70% identical to SEQ ID: 9, 10 or 18,
   b. Optionally co-expressing at least two casein proteins and one Fam20c kinase having an amino acid sequence at least 70% identical to SEQ ID: 23 in the host cell, and
   c. Culturing the host cell in a culture medium allowing for secretion of the casein proteins.
22. A method of increasing the secretion of casein from a recombinant *Kluyveromyces lactis* or *Pichia pastoris* host cell, comprising:
   a. Integrating a polynucleotide encoding at least two casein proteins into the host cell genome, wherein the polynucleotide is linked to the heterologous secretion signal pOst1-aMF having the SEQ ID: 24 and to a promoter, and wherein the polynucleotide encodes casein κ having an amino acid sequence 70% identical to SEQ ID: 14 in combination with casein αs1 having the amino acid sequence at least 70% identical to SEQ ID: 2, casein αs2 having the amino acid sequence at least 70% identical to SEQ ID: 6 and/or casein β having the amino acid sequence at least 70% identical to SEQ ID: 10.
   b. Co-expressing at least two casein proteins in the host cell, and
   c. Culturing the host cell in a culture medium allowing for secretion of the casein proteins.

### EXAMPLES

The following Examples illustrate the invention but are not to be construed as limiting the scope of the invention.

### Example 1: Production of recombinant K. lactis

Yeast strain: K. lactis from NEB (E1000S).

K. lactis vector:

| K lactis vector | Genomic integration site | Promoter | Terminator | Selection |
|---|---|---|---|---|
| pKLAC2 | LAC4 locus | LAC4 (inducible) | LAC4 TT | nitrogen auxotrophy (amdS selection) |

Co-expression combinations:

| K. lactis | Cassette 1 | | | Cassette 2 | | | Cassette 3 | | | Terminator |
|---|---|---|---|---|---|---|---|---|---|---|
| Combination no. | promoter | SS | ORF | promoter | SS | ORF | promoter | SS | ORF | |
| 1 | pLAC4 | aMF | aS1_m | pLAC4 | -- | -- | pLAC4 | -- | -- | LAC4 tt |
| 2 | | aMF | K_m | | -- | -- | | -- | -- | |
| 3 | | aMF | aS1_m | | aMF | K_m | | -- | -- | |
| 4 | | pOstl- aMF | K_m | | -- | -- | | -- | -- | |
| 5 | | aMF | aS1_m | | pOstl- aMF | K_m | | -- | -- | |
| 6 | | aMF | aS1 | | -- | hFAM20c | | -- | -- | |
| 7 | | aMF | K | | -- | hFAM20c | | -- | -- | |
| 8 | | pOstl- aMF | K | | -- | hFAM20c | | -- | -- | |
| 9 | | aMF | aS1 | | aMF | K | | -- | hFAM20c | |
| 10 | | aMF | aS1 | | pOstl- aMF | K | | -- | hFAM20c | |

Vector construction, yeast transformation, as well as expression tests are performed according to manufacturer's instructions.

Co-expression strategy in *K. lactis* similar to that in *Pichia,* except in case of (co-)expression of wild type casein(s), the hFAM20c kinase without secretion signal is integrated to the same genomic locus as the caseins. The expression of the kinase is inducible.

Every single casein (WT or phosphomimetic) will be cloned into a pKLAC2 vector. Each single casein has therefore its own pLAC4 promoter, which is needed for inducible expression and homologous recombination. The co-expression will be achieved by co-transformation of multiple linearized pKLAC2 vector. Multiple caseins will be integrated into the genomic LAC4 locus by homologous recombination. Compared to Pichia co-expression system, 2A peptide linker is not required in K. lactis.

### 1.1. K. lactis electroporation

Adapted from SANCHEZ et al. 1993 (M. Sánchez, F. J. Iglesias, C. Santamaría, and A. Domínguez, "Transformation of Kluyveromyces lactis by Electroporation," Appl Environ Microbiol, vol. 59, no. 7, pp. 2087-2092, Jul. 1993*)* and Wesolowski-Louvel 2011(Wésolowski-Louvel, "An efficient method to optimize Kluyveromyces lactis gene targeting," FEMS Yeast Res, vol. 11, no. 6, pp. 509-513, Sep. 2011*,)*
- K lactis overnight preculture was diluted to OD600 0.2 in 50 mL YPD and let grow to OD 0.8-1.0.
- 2. Cells were pelleted at 3000 g for 5 min at 4 °C, then washed with equal volume of ice cold dH2O.
- 3. Pelleted cells were resuspended in 2 mL of Pretreating buffer: YPD containing 25 mM DTT and 20 mM HEPES, pH 8.0.
- 4. Incubate at 30 °C for 30 min.
- 5. Pellet cells again and wash with 10 mL of ice-cold Electroporation buffer: 10 mM Tris-HCl pH 7.5, 270 mM sucrose, 1 mM MgCl2.
- 6. Resuspend pellet in 100 uL of ice-cold respective Electroporation buffer.
- 7. Mix 50 uL of electrocompetent cells with column purified Sacll-digested pKLAC2 vector(s) (1.0 ug each) + 1 uL of Salmon sperm ssDNA.
- 8. Electroporation program: 2 mm cuvette, 1.0 Kv. Measurements: 5.6-6.0 mS.
- 9. After the pulse, 1 mL of cold YPD was added to the cells and the suspension was transferred back to the culture tube and kept on ice for 5 min before incubation at 30 °C.
- 10. After 1 h recovered at 30 °C, plate 50 uL - 300 uL of each transformation recovery culture on YCB+ 5 mM acetamide plates.
- 11. Incubate plates at 30 °C for 3-5 days until 1-2 mm diameter colonies appear. * (Note: there will be a background of negative clones, as K. lactis contains a weakly active native acetamidase which will allow for minimal growth of untransformed cells on selection media.)
- 12. Colonies from YCB+5mM acetamide plates were then re-streaked on YCB + 5mM acetamide plates to isolate single colonies, prior to yeast colony PCR for genomic integration verification.

### 1.2 K. lactis colony PCR to check genomic integration

Single colonies were dissolved in 10 uL of sterile water, 1 uL of K lactis cell suspension was used as PCR template solution. The rest of the cells were used to make master plate, and liquid culture for glycerol stock and expression test.

Amplification of the gene of interest is carried out either primers included in the kit, or with casein gene specific primers.

### Example 2: Production of recombinant Pichia pastoris

Pichia strain: PichiaPink from ThermoFisher (A11150).

Pichia vectors:

| Pichia vector | Genomic integration site | Promoter | Terminator | Selection |
|---|---|---|---|---|
| pPINK-HC | AOX1 locus | AOX1 (inducible) | CYC1 TT | adenine auxotrophy |
| pGAPz-A | GAP1 locus | GAP (constitutive) | AOX1 TT | Zeocin resistance |

### Co-expression combinations

| P. pastoris | AOX1 locus | | | | | | | | | | GAP locus | | |
|---|---|---|---|---|---|---|---|---|---|---|---|---|---|
| | Cassette 1 | | | Cassette 2 | | | Cassette 3 | | | Termin ator | promo ter | ORF | Termi nator |
| Combi nation no. | promote r | SS | ORF | 2A | SS | ORF | 2A | SS | ORF | CYC1 tt/AOX 1 tt | | | |
| 1 | pAOX1/ pGAP | aMF | aS1_ m | | | | | | | | | | |
| 2 | | pOst1-aMF | K_m | | | | | | | | | | |
| 3 | | aMF | aS1_ m | T2A_ 1 | aMF | K_m | | | | | | | |
| 4 | | pOst1-aMF | K_m | | | | | | | | | | |
| 5 | | aMF | aS1_ m | T2A_ 1 | pOst1 -aMF | K_m | | | | | | | |
| 6 | | aMF | aS1 | | | | | | | | pGAP | hFA M20c | AOX 1 TT |
| 7 | | aMF | K | | | | | | | | | | |
| 8 | | aMF | aS1 | T2A_ 1 | | | | | | | | | |
| 9 | | pOst1-aMF | K | | aMF | K | | | | | | | |
| 10 | | aMF | aS1 | T2A_ 1 | pOst1 -aMF | K | | | | | | | |
| 11 | | a-amylase | aS1 | T2A_ 1 | aMF | beta | T2A _2 | pOst1 -aMF | K | | | | |
| 12 | | Killer protein | | | | | | | | | | | |
| 13 | | HSA | | | | | | | | | | | |

Vector construction, yeast transformation, as well as expression tests would be performed according known protocols.

To achieve the (co-)expression of wild type casein(s), the hFAM20c kinase is integrated into the GAP1 locus to achieve constitutive expression. Then single WT casein (without native signal peptide) or multiple WT caseins with 2A peptide linker(s) are cloned into the pPINK-HC vector. Linearized vector expressing single casein or multiple caseins with 2A linker(s) could be subsequently integrated into the AOX1 locus for inducible secretion. Different secretion signals will be tested to optimize the secretion process.

### pOst1-aMF

SEQ ID NO: 24

ATGAGGCAGGTTTGGTTCTCTTGGATTGTGGGATTGTTCCTATGTTTTTTCAACGTGTCTTCTGCTGCTCCAGTCAA

CACTACAACAGAAGATGAAACGGCACAAATTCCGGCTGAAGCTGTCATCGGTTACTCAGATTTAGAAGGGGATTT
CGATGTTGCTGTTTTGCCATTTTCCAACAGCACAAATAACGGGTTATTGTTTATAAATACTACTATTGCCAGCATTgc
tgCTAAAGAAGAAGGGGTATCTTTGGATAAAAGAGAGGCTGAAGCT

### Linker 2A peptide sequences

SEQ ID NO: 29/30
T2A_1
   AGAGCTGAGGGTAGAGGTTCTTTGCTTACTTGCGGTGACGTTGAGGAAAACCCAGGTCCA
T2A_2
   CGTGCCGAAGGACGTGGATCCCTTTTGACCTGCGGAGATGTCGAAGAGAATCCTGGACCT

In case of inducible secretion casein mutant(s), single mutant casein (without native signal peptide), or multiple mutant caseins with 2A peptide linker(s) could be integrated into AOX1 locus for inducible secretion. Different secretion signals will be tested to optimize the secretion process.

### 2.1 Pichia electroporation

- A 50-ml culture of Pichia strain (X-33, GS115, or PichiaPINK) was grown in YPD at 30°C with good aeration from OD 0.2 to an OD600 of 1-2.
- The culture was then supplemented with 1 ml of 1.0 M Na+-HEPES, pH 8.0 and 1 ml of 1.0 M DTT, and incubation was continued for an additional 15 min at 30 °C.
- The cells were transferred to a chilled 50-ml Falcon tube, centrifuged for 3 min at 4000 g at 4 °C, and resuspended in 50 ml ice-cold double-distilled H2O. This H2O wash step was repeated, followed by a wash with 20 ml cold 1.0 M sorbitol.
- Finally, the cells were resuspended in 200 ul cold 1.0 M sorbitol. A 80 uL aliquot of yeast cells was mixed with 0.1-1 ug DNA in 5 ul of a low-salt solution. This mixture was transferred to a chilled electroporation cuvette (0.2 cm gap) and pulsed with a Bio-Rad Gene Pulser set at 1.5 kV, 25 uF, 200 ohms (time constant 5 ms).
- The cell suspension was immediately diluted with 1 ml of cold YPDS media (YPD +1.0 M sorbitol) to the cuvette and mix by pipetting up and down.
- Incubate the cells in the cuvette at 24°C -30°C for at least 2 hours (up to 12 h) without shaking.
- Finally, the cells were spread on appropriate selection plates (may be supplemented with 1.0 M sorbitol), and the plates were incubated at 30 °C for 2-3 days until colonies appeared.

### 2.2 Pichia colony PCR to check genomic integration

Single colonies were dissolved in 10 uL of sterile water, 1 uL of K lactis cell suspension was used as PCR template solution. The rest of the cells were used to make master plate, and liquid culture for glycerol stock and expression test.

### - PichiaPINK strain with pPINK plasmids integration

Amplification of the gene of interest is carried out either with the 5' AOX1 forward primer (pPink-HC, pPink-LC, and pPinkα-HC) or the α-factor forward primer (pPinkα-HC only) paired with the 3' CYC1 reverse primer included in the kit.

### - X-33 strain with pGAP plasmids integration

Amplification of the gene of interest is carried out using the pGAP Forward and the AOX1 terminator reverse primer.

### - X-33 strain with pPINK plasmids integration

Amplification of the gene of interest is carried out using the pAOX1 Forward and the AOX1 terminator reverse primer.

### Example 3:

K. lactis strains baring genomic single insertion of a nucleic acid pOst1-aMF-Kappa casein encoding the amino acid of SEQ ID NO:16, or multiple insertions of aMF-Kappa casein were grown in YPD medium, before diluting to OD600 0.2 in induction medium, i.e., YP medium supplemented with 2 % of Galactose. The cultures were incubated in baffled or non-baffled shake flasks at 20 °C, with 120 or 240 rpm, for 72 h. Galactose feeding was added after 16-, 24-, 40- and 48-hours culture time. Culture supernatants were harvested and analysed by SDA-PAGE, followed by anti-Kappa casein Western blot. Kappa casein signals were quantified by comparison to the milk standard.

### Results

### • Glycosylation

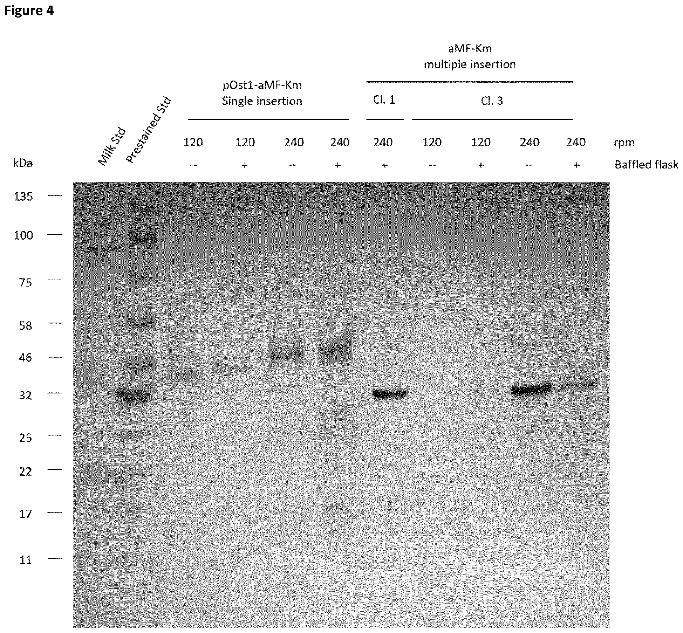

The Western blot analysis (Fig. 4) revealed that Kappa casein expressed and secreted from the pOst1-pOst1-aMF-Kappa casein construct migrate slower than the one from aMF-Kappa casein construct. This could be a result from higher level or uneven glycosylation pattern in the former construct.

### • Culture condition

In term of culture conditions, the combination of 20 °C incubation temperature (better than 30 °C, data not shown), 240 rpm shaking speed and the use of baffled shake flask seemed to give the best result.

### • Yield and clonal variation

Under the best culture conditions, the yield of the pOst1-aMF-Kappa casein from different clones are listed in the following table.

| Construct | Genomic integration copy number | Yield (g/L) |
|---|---|---|
| pOst1-aMF-Kappa Cl. 1 | Single copy | 0.23 |
| aMF-Kappa Cl. 1 | Multi copy (≥ 2) | 0.18* |
| aMF-Kappa Cl. 3 | Multi copy (≥ 2) | 0.26 |

| | | |
|---|---|---|
| * As shown in SDS-PAGE analysis (Fig. 3), aMF-Kappa casein Cl. 1 sample had only half of the amount of total protein loaded compared to all other samples. If the same amount of total protein is loaded, this clone may have higher Kappa mutant yield compared to aMF-Kappa casein Cl. 3. | | |

The amount of Kappa casein produced from pOst1-aMF and aMF secretion signal in the analysed clones are in the same range, i.e., about 0.25 g/L. Importantly, the pOst1-aMF-Kappa casein recombinant organism has single copy insertion, while the aMF-Kappa recombinant organism has multiple copy (≥ 2) insertion. Hence, we deduct that if we compare a multi-copy pOst1-aMF-Kappa recombinant organism vs a multi-copy aMF-Kappa recombinant organism, we should have better yield from the former construct.

## Claims

1. A recombinant host cell genetically engineered to express at least one casein protein, wherein the polynucleotide encoding the casein protein is integrated into the host cell genome or is maintained episomally and is linked to a heterologous secretion signal and to a promoter and is followed by a terminator sequence, optionally wherein the host cell is genetically engineered to produce at least two, at least three or at least 4 different casein proteins.

2. The recombinant host cell of claim 1, wherein the casein protein encoded by the polynucleotide is at least 70% identical to casein αs1 having the amino acid sequence of SEQ ID NO: 1, 2 or 17, casein αs2 having the amino acid sequence of SEQ ID NO: 5 or 6, casein β having the amino acid sequence of SEQ ID NO:9, 10 or 18 or casein κ having the amino acid sequence of SEQ ID NO: 13, 14 or 19, preferably wherein the host cell is genetically engineered to co-express casein κ having the amino acid sequence at least 70% identical to SEQ ID:13, 14 or 19 in combination with casein αs1 having the amino acid sequence at least 70% identical to SEQ ID: 1, 2 or 17, casein αs2 having the amino acid sequence at least 70% identical to SEQ ID: 5 or 6 and/or casein β having the amino acid sequence at least 70% identical to SEQ ID:9, 10 or 18.

3. The recombinant host cell of claims 1 or 2, wherein one or more the nucleotides encoding serine or threonine of the casein have been replaced with nucleotides encoding glutamic acid or aspartic acid.

4. The recombinant host cell of any one of claims 1 to 3, wherein the polynucleotide encoding the casein has a nucleotide sequence selected from the group consisting of SEQ ID NO: 3, 4, 7, 8, 11, 12, 15, 16, or 20 to 22.

5. The recombinant host cell of any one of claims 1 to 4, wherein the heterologous secretion signal is not a casein secretion signal.

6. The recombinant host cell of any one of the preceding claims, wherein the host cell is an organism, such as a bacterium, a yeast or a filamentous fungus, optionally wherein the organism is a bacterium preferably selected from the group consisting of *Escherichia coli* and *Bacillus subtilis,* a filamentous fungus selected from the group consisting of *Trichoderma reesei, Aspergillus oryzae* and *Aspergillus niger,* or a yeast cell preferably selected from the group of Kluyveromyces sp., Pichia sp., Saccharomyces sp., Tetrahymena sp., Yarrowia sp., Hansenula sp., Blastobotrys sp., Candida sp., Zygosaccharomyces sp., and Debaryomyces sp, preferably wherein the host cell is *Kluyveromyces lactis* or *Pichia pastoris.*

7. The recombinant host cell of claim 6, wherein the host cell is a yeast cell and the heterologous secretion signal is selected from the group consisting of pOst1-aMF having the SEQ ID: 24, aMF having the SEQ ID NO: 25 or 26 or Killer protein having SEQ ID NO: 27.

8. The recombinant host cell of claim 6 or 7, wherein the host cell is *Kluyveromyces lactis* or *Pichia pastoris,* preferably wherein in the promoter is SEQ ID NO: 28 or SEQ ID NO:37.

9. The recombinant host organism of any one of claims 6 to 8, wherein:
a. the host cell is *Kluyveromyces lactis* or *Pichia pastoris;*
b. the heterologous secretion signal is pOst1-aMF having the SEQ ID: 24; and
c. the host cell is genetically engineered to co-express casein κ having the amino acid sequence at least 70% identical to SEQ ID: 13, 14, or 19 in combination with casein αs1 having the amino acid sequence at least 70% identical to SEQ ID: 1, 2 or 17, casein αs2 having the amino acid sequence at least 70% identical to SEQ ID: 5 or 6 and/or casein β having the amino acid sequence at least 70% identical to SEQ ID:9, 10 or 18.

10. Use of a recombinant host cell of any one of claims 1 to 9 for manufacturing at least one casein protein of interest.

11. A method of producing at least one casein protein of interest, comprising the steps of:
a. Expressing a polynucleotide encoding at least one casein protein having an amino acid sequence 70% identical to SEQ ID NO: 1, 2, 5, 6, 9, 10, 13 or 14 linked to a secretion signal in a recombinant host cell according to any one of claims 1 to 15;
b. Culturing the host cell in a culture medium under conditions sufficient to allow for secretion of the at least one casein protein into the culture medium, optionally wherein:
c. the casein protein in step (a) is co-expressed with at least a second casein protein having an amino acid sequence at least 70% identical to any one of SEQ ID NO: 1, 2, 5, 6, 9, 10, 13 or 14 and/or co-expressed with a FAM20c kinase having an amino acid sequence at least 70% identical to SEQ ID: 23.

12. A casein protein produced by the method of claim 11 or having the amino acid sequence of SEQ ID: 2, 6, 10 or 14.

13. A micelle comprising one or several casein proteins according to claim 12.

14. A composition comprising a casein protein of claim 12 or a micelle of claim 13.

15. A method of increasing the secretion of casein from a recombinant *Kluyveromyces lactis* or *Pichia pastoris* host cell, comprising:
a. Integrating a polynucleotide encoding at least two casein proteins having at least 70% amino acid identity to SEQ ID NO: 1, 2, 5, 6, 9, 10, 13 or 14 and one Fam20c kinase having at least 70% amino acid identity to SEQ ID NO: 23 into the host cell genome, wherein the polynucleotide is linked to the heterologous secretion signal pOst1-aMF having the SEQ ID: 24 and to a promoter having SEQ ID NO:28, and wherein the polynucleotide encodes casein κ having an amino acid sequence 70% identical to SEQ ID: 13, 14, or 19 in combination with casein αs1 having the amino acid sequence at least 70% identical to SEQ ID: 1, 2 or 17, casein αs2 having the amino acid sequence at least 70% identical to SEQ ID: 5 or 6 and/or casein β having the amino acid sequence at least 70% identical to SEQ ID: 9, 10 or 18,
b. Optionally co-expressing at least two casein proteins and one Fam20c kinase having an amino acid sequence at least 70% identical to SEQ ID: 23 in the host cell, and
c. Culturing the host cell in a culture medium allowing for secretion of the casein proteins.
